# EUROPEAN PATENT APPLICATION

(11) **EP 4 282 423 A1**
(43) Date of publication of application: **29.11.2023**
(21) Application number: 21921457.4
(22) Date of filing: 29.11.2021
(51) Int. Cl.: A61K 35/28, A61K 38/16, A61K 38/18, A61P 29/00, C12N 5/0775

(54) **PHARMACEUTICAL COMPOSITION FOR PREVENTION OR TREATMENT OF INFLAMMATORY DISEASE OR PAIN, COMPRISING MESENCHYMAL STEM CELLS EXPRESSING PTX-3, TIMP1 AND BDNF AS ACTIVE INGREDIENT**

(30) Priority: 22.01.2021 KR 20210009736
(71) Applicant: Medipost Co., Ltd., Bundang-gu, Seongnam-si Gyeonggi-do 13494 (KR)
(72) Inventor: YANG, Yoon Sun, Seongnam-si, Gyeonggi-do 13494 (KR); OH, Wonil, Seongnam-si, Gyeonggi-do 13494 (KR); CHOI, Soo Jin, Seongnam-si, Gyeonggi-do 13494 (KR); JIN, Hye Jin, Seongnam-si, Gyeonggi-do 13494 (KR); BAE, Yun Kyung, Seongnam-si, Gyeonggi-do 13494 (KR); LEE, Minju, Seongnam-si, Gyeonggi-do 13494 (KR)
(74) Representative: Dehns
(86) International application number: PCT/KR2021/017772
(87) International publication number: WO 2022/158698

(57) **Abstract**

The present invention relates to a pharmaceutical composition for the prevention or treatment of inflammatory disease, comprising mesenchymal stem cells expressing PTX-3, TIMP1, and BDNF as an active ingredient. The mesenchymal stem cells expressing PTX-3, TIMP1, BDNF, and/or HLA-A2, according to the present invention, may inhibit an inflammatory response by reducing the expressions of the inflammatory cytokines IL-1b, IL-6, IL-8, and TNF-a, and increasing the expressions of the anti-inflammatory cytokines IL-10 and arginase-1 (ARG-1), and may relieve pain caused by osteoarthritis when administered in an osteoarthritis-induced rat. Thus, the mesenchymal stem cells expressing PTX-3, TIMP1, and BDNF of the present invention may be usefully employed in the prevention, alleviation, or treatment of inflammatory disease, pain, and pain cuased by inflammation.

## Description

### Technical Field

The present invention relates to a pharmaceutical composition for prevention or treatment of an inflammatory disease or pain, comprising mesenchymal stem cells as an active ingredient. More specifically, the present invention relates to a pharmaceutical composition for prevention or treatment of an inflammatory disease or pain, comprising, as an active ingredient, mesenchymal stem cells that exhibit overexpression or increased expression of one or more proteins selected from the group consisting of PTX-3, TIMP1, and BDNF.

### Background Art

Inflammation is an immune response to harmful stimuli, including external pathogens, toxins, and damaged cells. Such inflammation shows signs such as swelling, pain, or loss in tissue stiffness, function, and movement. In particular, inflammation occurring in the joint causes pain, and impaired activities of daily living and impaired physical mobility, thereby decreasing quality of life. The global market size for drugs used to treat symptoms related to inflammation is about $106.1 billion as of 2020, which is large enough to match the market size for anticancer drugs. Among these, therapeutic agents for arthritis accounted for 42.41% of the global import sales in 2019, and a rapid growth rate of 6.60% is expected therefor over the next few years from 2020 to 2026.

In the early stages of osteoarthritis, inflammatory cytokines (IL-6, TNF-a, IL-8, and the like) are secreted in the synovial membrane and cartilage tissue due to external stimuli. The thus secreted cytokines continuously induce inflammation in joint tissue while causing pain, and activate cartilage degradation enzymes and bone destruction factors, which results in joint destruction and ultimately causes joint deformity (Piotr Wojdasiewicz et al., Mediators Inflamm. 2014: 561459). Nonsteroidal anti-inflammatory drugs (NSAIDs) are mainly used as drugs for treating such arthritis. Among these, Humira (AbbVie), which is an anti-TNF agent, recorded the highest sale as a single drug in the United States in 2018. Such drugs may help reduce daily pain; however, these drugs have adverse effects such as gastrointestinal disorder, and this makes it difficult to expect long-term effects. Therefore, it is necessary to develop a therapeutic agent that affects the mechanism of occurrence of arthritis inflammation and thus enables fundamental treatment.

To solve this problem, various research and development activities for stem cell therapeutic agents are being conducted. Mesenchymal stem cells can be isolated and cultured from various tissues such as bone marrow, umbilical cord blood, and adipose tissue, and can be differentiated into cartilage tissue. Thus, these cells are used as a fundamental therapeutic agent for arthritis. Proteins secreted by mesenchymal stem cells have been reported to be effective in treatment of various diseases and tissue regeneration due to their anti-apoptotic, immunosuppressive, and anti-inflammatory effects, and the like (Mancuso et al., Front Bioeng Biotechnol. 2019 Jan 29;7:9., D. S. Jevotovsky et al., Osteoarthritis Cartilage. 2018 Jun;26(6):711-729). In particular, it has been reported that the anti-inflammatory effect of mesenchymal stem cells can regulate polarization of macrophages as well as secretion of anti-inflammatory cytokines, thereby fundamentally inhibiting occurrence of inflammatory responses (Meng Sun et al., J Immunol Res. 2019 Jun 19;2019:7059680).

The above-described therapeutic effects caused by mesenchymal stem cells may be affected by donor, tissue from which these cells are derived, isolation method, culture method, and the like. To solve this problem, it is necessary to find out characteristics that make it possible to select cells with therapeutic effects.

### [Citation List]

### [Non-Patent Literature]

(Non-Patent Literature 1) Piotr Wojdasiewicz et al., Mediators Inflamm., 2014: 561459
(Non-Patent Literature 2) Mancuso et al., Front Bioeng Biotechnol., 2019 Jan 29;7:9
(Non-Patent Literature 3) D. S. Jevotovsky et al., Osteoarthritis Cartilage. 2018 Jun;26(6):711-729
(Non-Patent Literature 4) Meng Sun et al., J Immunol Res. 2019 Jun 19;2019:7059680

### Disclosure of Invention

### Technical Problem

As a result of studies to obtain mesenchymal stem cells that are effective in prevention or treatment of an inflammatory disease, the present inventors have identified that mesenchymal stem cells exhibit increased expression of specific proteins under an inflammatory condition, and that the mesenchymal stem cells expressing such proteins have excellent anti-inflammatory and pain reduction effects, thereby completing the present invention.

### Solution to Problem

To solve the above-mentioned problem, in an aspect of the present invention, there is provided a pharmaceutical composition for prevention or treatment of an inflammatory disease or pain, comprising, as an active ingredient, mesenchymal stem cells expressing one or more proteins selected from the group consisting of PTX-3, TIMP1, and BDNF.

In another aspect of the present invention, there is provided a method for obtaining mesenchymal stem cells with improved anti-inflammatory or pain reduction efficacy, the method comprising a step of stimulating mesenchymal stem cells so that the mesenchymal stem cells exhibit increased expression of one or more proteins selected from the group consisting of PTX-3, TIMP1, and BDNF.

In another aspect of the present invention, there is provided a method for preventing or treating an inflammatory disease or pain, comprising a step of administering the pharmaceutical composition to an individual.

In another aspect of the present invention, there is provided a use of the pharmaceutical composition for preventing or treating an inflammatory disease or pain.

In another aspect of the present invention, there is provided a use of the pharmaceutical composition for preparing a medicament for preventing or treating an inflammatory disease or pain.

### Advantageous Effects of Invention

The mesenchymal stem cells expressing PTX-3, TIMP1, and BDNF according to the present invention decrease expression of IL-1b, IL-6, IL-8, and TNF-a, which are inflammatory cytokines, and increase expression of IL-10 and arginase-1 (ARG-1), which are anti-inflammatory cytokines, so that an inflammatory response can be suppressed; and the mesenchymal stem cells can alleviate pain caused by osteoarthritis in a case of being administered to an osteoarthritis-induced rat. Accordingly, the mesenchymal stem cells expressing PTX-3, TIMP1, and BDNF of the present invention can be effectively used for prevention, alleviation, or treatment of an inflammatory disease, pain, and inflammation-induced pain.

### Brief Description of Drawings

FIG. 1 illustrates graphs showing increased rates in expression level of PTX-3, TIMP1, and BDNF in a case where SMUP-Cell is co-cultured with a macrophage inflammation model obtained by treating RAW264.7 cell line with lipopolysaccharide (LPS) so that an inflammatory response is induced, as compared with a case where SMUP-cell is cultured alone.
FIG. 2 illustrates results obtained by identifying expression of HLA-A2 in two types of SMUP-Cell co-cultured with a macrophage inflammation model.
FIG. 3 illustrates graphs showing expression levels of IL-6, TNF-a, and IL-10 in a case where SMUP-Cell expressing PTX-3, TIMP1, and BDNF is co-cultured with a macrophage inflammation model.
FIG. 4 illustrates graphs showing increased rates in expression level of PTX-3, TIMP1, and BDNF in a case where SMUP-Cell is co-cultured with a chondrocyte inflammation model obtained by treating SW1353 cell line with IL-1b so that an inflammatory response is induced.
FIG. 5 illustrates graphs showing expression levels of IL-1b, IL-8, and IL-6 in a case where each of three types of SMUP-Cell is co-cultured with a chondrocyte inflammation model.
FIG. 6 illustrates graphs showing expression levels of TNF-a and arginase-1 (ARG-1) in a case where SMUP-Cell, SMUP-Cell C-siR, or SMUP-Cell PTX-3 siR is co-cultured with a macrophage inflammation model.
FIG. 7 illustrates photographs showing stained CD11b (macrophage M1 marker) and CD206 (macrophage M2 marker), and proportions of stained cells, in a case where SMUP-Cell, SMUP-Cell C-siR, or SMUP-Cell PTX-3 siR is co-cultured with a macrophage inflammation model.
FIG. 8 illustrates graphs showing expression levels of IL-1b, IL-6, IL-8, and TNF-a in chondrocytes in a case where SMUP-Cell, SMUP-Cell C-siR, or SMUP-Cell PTX-3 siR is co-cultured with a chondrocyte inflammation model.
FIG. 9 illustrates a graph showing an expression level of PTX-3 in SMUP-Cell, SMUP-Cell C-siR, SMUP-Cell PTX-3 siR, or human umbilical vein endothelial cells (HUVEC).

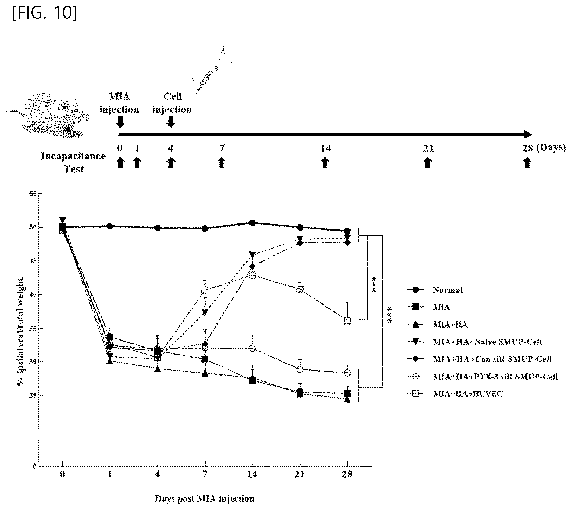

FIG. 10 illustrates a graph showing results obtained by injecting, into a rat in which arthritis pain has been induced by injection of monoiodoacetate (MIA), hyaluronic acid (HA) and SMUP-Cell, SMUP-Cell C-siR, SMUP-Cell PTX-3 siR, or HUVEC together, and performing an incapacitance test to measure weight bearing asymmetry.
FIG. 11 illustrates graphs showing expression levels of IL-1b, IL-6, IL-8, and TNF-a in chondrocytes in a case where SMUP-Cell, SMUP-Cell C-vec, or SMUP-Cell TIMP1 KO is co-cultured with a chondrocyte inflammation model.
FIG. 12 illustrates graphs showing expression levels of IL-6 and TNF-a in macrophages in a case where SMUP-Cell, SMUP-Cell C-siR, or SMUP-Cell BDNF siR is co-cultured with a macrophage inflammation model.
FIG. 13 illustrates a graph showing results obtained by identifying changes in inflammatory cytokine level in SMUP-Cell, SMUP-Cell C-vec, and PTX-3-overexpressing stem cells (SMUP-Cell-PTX3 OE).

### Best Mode for Carrying out the Invention

Hereinafter, the present invention will be described in more detail.

In an aspect of the present invention, there is provided a pharmaceutical composition for prevention or treatment of an inflammatory disease or pain, comprising, as an active ingredient, mesenchymal stem cells expressing one or more proteins selected from the group consisting of PTX-3, TIMP1, and BDNF. Specifically, the pharmaceutical composition may comprise, as the active ingredient, mesenchymal stem cells that exhibit overexpression or increased expression of one or more proteins selected from the group consisting of PTX-3, TIMP1, and BDNF.

The PTX-3 (pentraxin 3) refers to a member of the pentraxin superfamily characterized by cyclic multimeric structure. The PTX-3 is rapidly produced and released by several cell types, in particular by mononuclear phagocytes, dendritic cells (DCs), fibroblasts, and endothelial cells, in response to primary inflammatory signals. In addition, the PTX-3 activates the classical pathway of complement activation and promotes antigen recognition by macrophages and dendritic cells.

The TIMP1 (tissue inhibitor matrix metalloproteinase 1), which is a glycoprotein expressed in several tissues, refers to an endogenous inhibitor that regulates the action of matrix metalloproteinase (MMP). Specifically, the TIMP1 is a member of the TIMP family and is a natural inhibitor of matrix metalloproteinases that are a group of peptidases involved in degradation of the extracellular matrix. In addition to its inhibitory role against known matrix metalloproteinases, the TIMP1 is capable of promoting cell proliferation in a wide range of cell types, and may also have an anti-apoptotic function.

The BDNF (brain-derived neurotrophic factor) refers to a neurotrophin distributed in the brain. It is known that the BDNF promotes the growth of neurons and regulates the synthesis, metabolism, and release of neurotransmitters, and the activity of neurons.

The mesenchymal stem cells may express PTX-3, TIMP1, or BDNF. In addition, the mesenchymal stem cells may express PTX-3 and TIMP1; PTX-3 and BDNF; or TIMP 1 and BDNF. Furthermore, the mesenchymal stem cells may express PTX-3, TIMP1, and BDNF.

The present invention is based on the results that mesenchymal stem cells, which express or exhibit increased expression of one or more proteins selected from the group consisting of PTX-3, TIMP1, and BDNF, have excellent inflammation inhibition or pain reduction capacity.

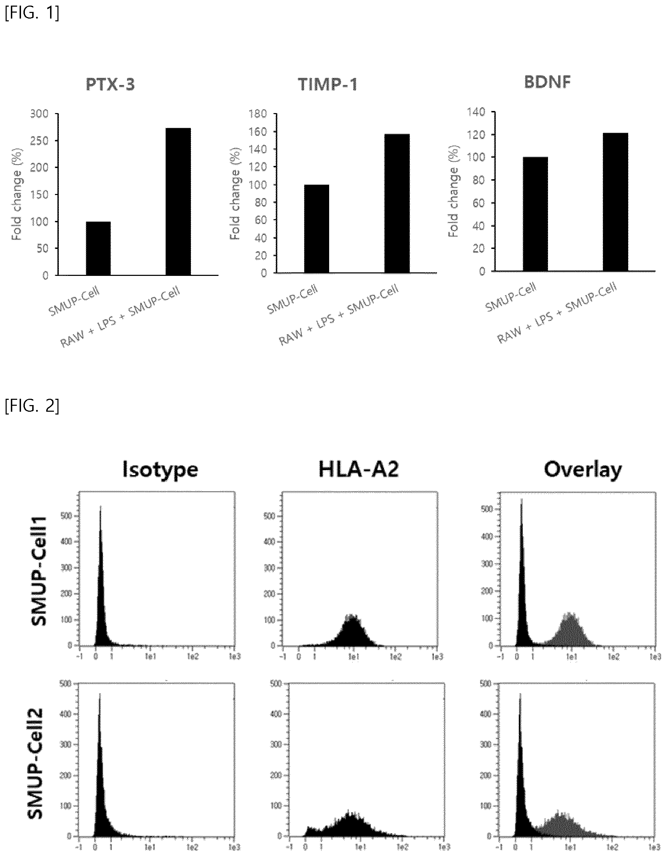

Specifically, in an embodiment of the present invention, in order to allow mesenchymal stem cells to be cultured under an inflammatory condition, SMUP-Cell at 2 × 10⁵ cells was co-cultured with a macrophage inflammation model prepared by subjecting RAW264.7 cell line, which is a mouse macrophage cell line, to treatment with 1 µg/ml of lipopolysaccharide (LPS). Then, changes in expression level of PTX-3, TIMP1, and BDNF in the medium after co-culture were checked. Furthermore, it was identified that the mesenchymal stem cells expressing any one selected from the group consisting of PTX-3, TIMP1, BDNF, and combinations thereof decreased expression of IL-1b, IL-6, IL-8, and TNF-a, which are inflammatory cytokines, and increased expression of IL-10 and arginase-1 (ARG-1), which are anti-inflammatory cytokines, so that an inflammatory response was suppressed (FIG. 1, FIG. 3, FIG. 5, FIG. 6, FIG. 8, FIG. 11, and FIG. 12).

The mesenchymal stem cells may additionally express HLA-A2. In an embodiment of the present invention, it was identified that the mesenchymal stem cells expressing any one selected from the group consisting of the PTX-3, TIMP1, BDNF, and combinations thereof additionally express HLA-A2 (FIG. 2).

The mesenchymal stem cells may express PTX-3 and HLA-A2, TIMP1 and HLA-A2, or BDNF and HLA-A2. In addition, the mesenchymal stem cells may express PTX-3, TIMP1, and HLA-A2; PTX-3, BDNF, and HLA-A2; or TIMP1, BDNF, and HLA-A2. Furthermore, the mesenchymal stem cells may express PTX-3, TIMP1, BDNF, and HLA-A2.

The HLA-A2 is one of serotypes that belong to the human leukocyte antigen (HLA)-A serotype group, and the HLA is a glycoprotein molecule encoded by a human major histocompatibility complex (MHC) gene. The HLA-A is expressed on the cell surface of all nucleated cells and platelets, and functions to cause cytotoxic T cells to recognize antigens so that the cytotoxic T cells recognize and eliminate virus-infected cells or tumor cells.

The mesenchymal stem cells may overexpress one or more proteins selected from the group consisting of PTX-3, TIMP1, and BDNF. The overexpression may be achieved through genetic manipulation, and specifically, the overexpression may be achieved by transformation using a vector loaded with a gene encoding any one selected from the group consisting of PTX-3, TIMP1, BDNF, and combinations thereof.

The mesenchymal stem cells may exhibit an increased expression level of any one selected from the group consisting of PTX-3, TIMP1, BDNF, and combinations thereof in a case of being subjected to inflammatory stimulation.

In the present invention, the inflammatory stimulation may mean exposure to an inflammatory environment that mimics an inflammatory response *in vivo.* The inflammatory stimulation may include, but is not limited to, stimulation caused by substances secreted by inflammatory cells, such as cytokines, exposure to an *in vitro* or animal inflammation model, or co-culture with inflammation-induced cells. In addition, the inflammatory condition or inflammatory environment may be an affected area where inflammation has occurred.

The mesenchymal stem cells may be prepared by a method including the following steps of:
(1) isolating mesenchymal stem cells having a size of 10 µm or lower;
(2) culturing the isolated mesenchymal stem cells in a culture medium containing calcium under a hypoxic condition of 2% to 5% oxygen; and
(3) subjecting the cultured mesenchymal stem cells to inflammatory stimulation.

In the above-described method, the isolated mesenchymal stem cells having a size of 10 µm or lower have superior proliferation capacity to mesenchymal stem cells having other sizes, and such mesenchymal stem cells may exhibit further improved proliferation capacity and differentiation capacity in a case of being cultured in a medium containing calcium under a hypoxic condition.

In the above-described method, the calcium may be contained in the culture medium at a concentration of 1.5 mM to 3.8 mM. Specifically, in the above-described method, the calcium may be contained in the culture medium at a concentration of 1.5 mM to 2.0 mM.

The culture may be performed in a conventionally known manner. For example, the mesenchymal stem cells may be cultured using a three-dimensional bioreactor (or spinner) or cultured in such a manner that a cell adhesive material is coated on a common adhesive container and a 3D carrier. In addition, the mesenchymal stem cells can be cultured using a 3D carrier.

The mesenchymal stem cells may be derived from umbilical cord blood, bone marrow, fat, muscle, skin, umbilical cord, amniotic fluid, or tooth. Specifically, the mesenchymal stem cells may be derived from umbilical cord blood.

For administration route and dosage of the pharmaceutical composition, administration to a subject may be performed in various ways and amounts depending on the subject's condition and the presence or absence of adverse effects, and the optimal administration method and dosage may be selected in an appropriate range by a person skilled in the art. In addition, the pharmaceutical composition may be administered in combination with other drugs or physiologically active substances whose therapeutic effect is known for the disease to be treated, or may be formulated in the form of a combination formulation with other drugs.

The pharmaceutical composition is a type of cell therapeutic agents, and may further comprise a pharmaceutically acceptable carrier. The carrier may be one commonly used in the preparation of medicines, and examples thereof may include lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, and mineral oil.

In addition, the pharmaceutical composition of the present invention may further comprise a pharmaceutically acceptable additive selected from the group consisting of a lubricant, a wetting agent, a sweetening agent, a flavoring agent, an emulsifying agent, a suspending agent, a preservative, and combinations thereof.

Based on the total weight of the pharmaceutical composition of the present invention, the carrier may be contained in an amount of about 1% to about 99.99% by weight, preferably about 90% to about 99.99% by weight, and the pharmaceutically acceptable additive may be contained in an amount of about 0.1% to about 20% by weight.

The pharmaceutical composition may be prepared in a unit dosage form by being formulated with a pharmaceutically acceptable carrier or excipient or may be prepared in a form of being placed in a multi-dose container, according to conventional methods. Here, the formulation may be in the form of a solution, a suspension, a syrup, or an emulsion, which is in oil or aqueous medium, or may be in the form of extracts, powders, granules, or capsules. The formulation may additionally comprise a dispersing or stabilizing agent.

The inflammatory disease refers to a disease accompanied by inflammation. Specifically, the inflammatory disease may be osteoarthritis, rheumatoid arthritis, atopy, asthma, allergic rhinitis, Alzheimer's disease, graft versus host disease (GVHD), diabetic nephropathy, Crohn's disease, inflammatory bowel disease, post-transplantation rejection, bronchopulmonary dysplasia (BPD), or chronic obstructive pulmonary disease (COPD). The pain may be inflammatory pain, and the inflammatory pain may be arthritis pain.

The individual to which the pharmaceutical composition is administered may be a mammal, specifically a human. For administration route and dosage of the pharmaceutical composition, administration to a subject may be performed in various ways and amounts depending on the subject's condition and the presence or absence of adverse effects, and the optimal administration method and dosage may be selected in an appropriate range by a person skilled in the art. In addition, the pharmaceutical composition may be administered in combination with other drugs or physiologically active substances whose therapeutic effect is known for the inflammatory disease to be treated, or may be formulated in the form of a combination formulation with other drugs.

In a case where the pharmaceutical composition is administered parenterally, examples thereof include subcutaneous, ocular, intraperitoneal, intramuscular, oral, rectal, intraorbital, intracerebral, intracranial, intraspinal, intraventricular, intrathecal, intranasal, intravenous, and intra-articular administration. Specifically, the pharmaceutical composition may be administered to an intra-articular site, a joint injury site, or a joint's medial and/or lateral compartment, in which the joint may be a knee, finger, or toe joint. The "intra-articular" administration may include "intra-articular cavity" administration.

The administration may be administered once or more times, or 1 to 3 times, specifically 3 times. In a case of repeated administration, administration may be performed at intervals of 1 to 56 days, 7 to 49 days, 14 to 42 days, or 21 to 35 days. Preferably, the administration may be performed at intervals of 28 days. For high doses, the administration may be performed several times a day.

A dosage of the mesenchymal stem cells may be 1 × 10⁵ cells/individual to 5×10⁷ cells/individual.

In another aspect of the present invention, there is provided a method for obtaining mesenchymal stem cells with improved anti-inflammatory or pain reduction efficacy, the method comprising a step of stimulating mesenchymal stem cells so that the mesenchymal stem cells exhibit increased expression of one or more proteins selected from the group consisting of PTX-3, TIMP1, and BDNF. The mesenchymal stem cells are as described above for the pharmaceutical composition.

The stimulating step may include a step of exposing the mesenchymal stem cells to an inflammatory environment. Specifically, the step of exposing the mesenchymal stem cells to an inflammatory environment may include, but is not limited to, stimulation caused by substances secreted by inflammatory cells, such as cytokines, co-culture with inflammation-induced cells, or exposure to an *in vitro* or animal inflammation model that mimics an inflammatory environment *in vivo.*

The inflammation-induced cells may be cells in which inflammation has been induced by treatment with lipopolysaccharide (LPS), a mitogen, a chemokine or a cytokine.

The cells may be any one selected from the group consisting of somatic cells, germ cells, and a combination thereof. The somatic cells may be any one selected from the group consisting of muscle cells, hepatocytes, neurons, fibroblasts, epithelial cells, adipocytes, bone cells, white blood cells, lymphocytes, platelets or mucosal cells, and combinations thereof. The germ cells may be any one selected from the group consisting of sperm, egg, and a combination thereof.

The mesenchymal stem cells may be prepared, before the stimulating step, by a method including the following steps of:
(1) isolating mesenchymal stem cells having a size of 10 µm or lower; and
(2) culturing the isolated mesenchymal stem cells in a culture medium containing calcium under a hypoxic condition of 2% to 5% oxygen.

In the above-described method, the calcium may be contained in the culture medium at a concentration of 1.5 mM to 3.8 mM. Specifically, in the above-described method, the calcium may be contained in the culture medium at a concentration of 1.5 mM to 2.0 mM.

Steps (1) and (2) are as described above for the pharmaceutical composition.

In yet another aspect of the present invention, there is provided a method for preventing or treating an inflammatory disease or pain, comprising a step of administering the pharmaceutical composition to an individual.

The individual may be a mammal, including a human, and may be a non-human animal. The term "non-human animal" refers to any vertebrate, and may include mammals and non-mammals, such as non-human primates, sheep, dogs, cats, horses, cows, chickens, amphibians, and reptiles.

The pharmaceutical composition may be appropriately administered to an individual, if necessary, according to an administration method, an administration route, and a dosage, which are conventionally used in the art. As an example of the administration route, parenteral administration may be mentioned. In addition, appropriate dosage and number of administrations may be selected according to methods known in the art; and the dosage and number of administrations for the actually administered composition may be appropriately determined depending on various factors such as type of symptom to be prevented or treated, administration route, sex, health condition, diet, individual's age and weight, and severity of disease.

In another aspect of the present invention, there is provided a use of the pharmaceutical composition for preventing or treating an inflammatory disease or pain.

In another aspect of the present invention, there is provided a use of the pharmaceutical composition for preparing a medicament for preventing or treating an inflammatory disease or pain.

### Mode for the Invention

Hereinafter, the present invention will be described in more detail by way of the following examples. However, the following examples are for illustrative purposes only, and the scope of the present invention is not limited thereto.

### Preparation Example 1. Preparation of mesenchymal stem cells (SMUP-CELL)

First, to prepare mesenchymal stem cells (SMUP-CELL), mononuclear cells were isolated from allogeneic umbilical cord blood using Ficoll-Plaque, and then dispensed at a concentration of 5×10⁶ to 1×10⁶ cells/cm². Culture was performed for up to 21 days in an incubator at 37°C, 5% CO₂, and 3% O₂. Then, the cells having a size of 10 µm or lower were isolated and dispensed at a concentration of 500 to 3,000 cells/cm². Culture was performed in α-MEM medium (SH30265.02, Hyclone) containing 5% to 30% fetal calf serum, which was supplemented with calcium at a concentration of 1.5 to 3.8 mM, for up to 7 days under a hypoxic (2% to 5%) condition.

### Example 1. Identification of anti-inflammatory effect of mesenchymal stem cells in macrophage inflammation model

### Example 1.1. Identification of increased expression of PTX-3, TIMP1, and BDNF in mesenchymal stem cells under inflammatory condition

A macrophage inflammation model was prepared by subjecting RAW264.7 cell line at 1×10⁵ cells, which is a mouse macrophage cell line, to treatment with 1 µg/ml of lipopolysaccharide (LPS). SMUP-Cell at 2×10⁵ cells was co-cultured with the macrophage inflammation model, and then analysis was performed. This was compared and analyzed with a case where SMUP-Cell was cultured alone. Here, as the basic culture medium, RPMI (22400, Gibco) was used to perform culture.

Specifically, in the medium, in which SMUP-Cell was cultured alone, and the medium, in which SMUP-Cell was co-cultured with the macrophage inflammation model, expression levels of PTX-3, TIMP1, and BDNF proteins were analyzed by human PTX-3 ELISA kit (DPTX30B, R&D Systems), human TIMP1 ELISA kit (DTM100, R&D Systems), and human BDNF ELISA kit (DBD00, R&D Systems). Here, the expression level of each protein measured in the medium, in which SMUP-Cell was cultured alone, was set to 100. With respect to this expression level, the expression level of each protein measured in the medium, in which SMUP-Cell was co-cultured with the macrophage inflammation model, was converted and comparison was performed.

As a result, it was identified that expression levels of PTX-3, TIMP1, and BDNF proteins increased in the medium in which SMUP-Cell was co-cultured with the macrophage inflammation model (FIG. 1).

### Example 1.2. Identification of expression of HLA-A2 in mesenchymal stem cells under inflammatory condition

SMUP-Cell at 2×10⁵ cells was co-cultured with the macrophage inflammation model prepared in Example 1.1 using the same culture condition (RPMI (22400, Gibco)), and then expression of HLA-A2 in SMUP-Cell was checked.

Specifically, the co-cultured SMUP-Cell was treated with trypsin to be separated into single cells, and then washed twice with PBS solution. Then, the washed SMUP-Cell was reacted with an anti-HLA-A2 antibody labeled with PE, and this was set as an experimental group. As a negative control, an isotype control-PE was used for the reaction. In the experimental group and the negative control, expression of HLA-A2 was checked using FACS. Here, for the co-cultured SMUP-Cell, two different types of donors were used.

As a result, fluorescence signals were measured in the co-cultured SMUP-Cell 1 and SMUP-Cell 2, each of which was the experimental group. From this, it was identified that HLA-A2 was expressed in SMUP-Cell 1 and SMUP-Cell 2, each of which was co-cultured with the macrophage inflammation model (FIG. 2).

### Example 1.3. Identification of anti-inflammatory effect of mesenchymal stem cells expressing PTX-3, TIMP1, BDNF, and HLA-A2

The media after co-culture in Example 1.2 were obtained, and expression levels of inflammatory cytokines therein were checked through ELISA analysis for mouse IL-6 and mouse TNF-a. Specifically, the analysis was performed according to the manufacturer's protocol using Mouse IL-6 ELISA kit (DY406, R&D Systems) and Mouse TNF-a ELISA kit (DY410, R&D Systems). Each culture medium sample after co-culture was diluted between 1 and 50, and analyzed. Then, an OD value for the reaction solution was measured using a VERSA max microplate reader (Molecular Device). In addition, an expression level of an anti-inflammatory cytokine was checked through ELISA analysis (DY417, R&D Systems) for mouse IL-10. Here, even for the medium, in which non-inflammation-induced RAW264.7 cell line was cultured alone, and the medium, in which the macrophage inflammation model was cultured, expression levels of mouse IL-6, mouse TNF-a, and mouse IL-10 were compared and analyzed through ELISA analysis similarly.

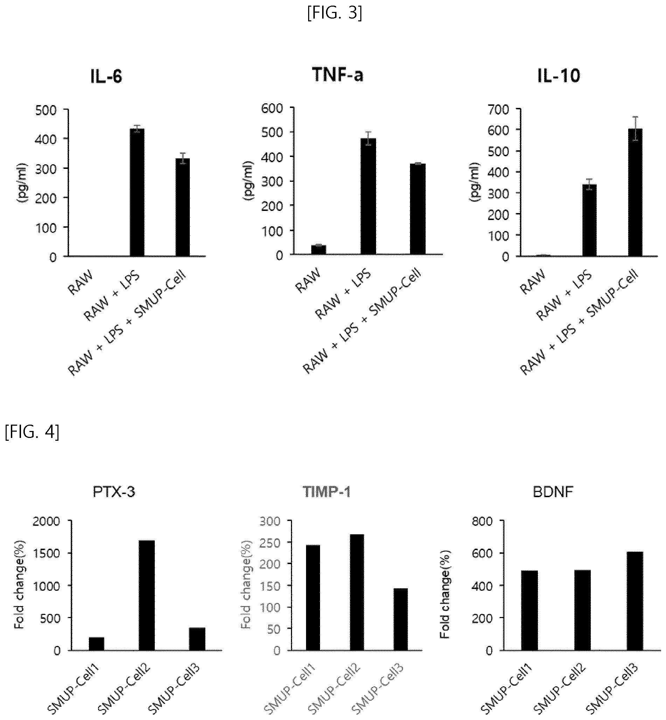

As a result, it was identified that IL-6, TNF-a, and IL-10 all increased in the medium, in which the macrophage inflammation model was cultured, as compared with the medium in which the RAW264.7 cell line was cultured alone. On the contrary, it was identified that in the medium, in which SMUP-Cell was co-cultured with the macrophage inflammation model, expression levels of IL-6 and TNF-a decreased, and an expression level of IL-10 increased (FIG. 3).

### Example 2. Identification of anti-inflammatory effect of mesenchymal stem cells in chondrocyte inflammation model

### Example 2.1. Identification of increased expression of PTX-3, TIMP1, and BDNF in mesenchymal stem cells under inflammatory condition

A chondrocyte inflammation model was prepared by subjecting SW1353 cell line (Chon) at 1×10⁵ cells, which is a human chondrocyte cell line, to treatment with 10 ng/ml of IL-1b. Then, SMUP-Cell at 1×10⁵ cells was co-cultured with the prepared chondrocyte inflammation model, and then analysis was performed. Here, expression levels of PTX-3, TIMP 1, and BDNF were measured in the same manner as in Example 1.1, and compared and analyzed with a case where SMUP-Cell was cultured alone. For SMUP-Cell, three different types received from three different donors were used. For the respective three types of SMUP-Cell, further increased rates in the expression levels in the media after co-culture were graphically expressed. Numerical calculation was performed by '(Chon + IL-1b + SMUP-Cell) culture medium / SMUP-Cell alone culture medium * 100', and only the calculated values were graphically expressed.

As a result, PTX-3, TIMP1, and BDNF were hardly expressed in a case where chondrocytes were cultured alone or in a case where the chondrocyte inflammation model was cultured. On the contrary, it was identified that expression levels of PTX-3, TIMP1, and BDNF increased in a case where SMUP-Cell was co-cultured with the chondrocyte inflammation model (FIG. 4).

### Example 2.2. Identification of anti-inflammatory effect of mesenchymal stem cells expressing PTX-3, TIMP1, and BDNF

It was identified whether co-culture of SMUP-Cell at 1×10⁵ cells with the chondrocyte inflammation model prepared in Example 2.1 could decrease inflammation in the chondrocytes. Here, for SMUP-Cell, three different types received from three different donors were used. The experiment was conducted using the groups divided as follows: 1) group (Chon) in which chondrocytes were cultured alone, 2) group (Chon + IL-1b) in which the chondrocyte inflammation model was cultured, and 3) group in which each of the three types of SMUP-Cell was co-cultured with the chondrocyte inflammation model.

The chondrocytes cultured in each of the groups 1) to 3) were treated with trypsin to be separated into single cells, and then mRNA was isolated therefrom to analyze expression levels of IL-1b, IL-8, and IL-6, which are inflammatory cytokines, by qPCR. Then, GAPDH was used to perform comparative analysis with normalized values. qPCR was performed as follows. The cells were treated with TRIzol reagent (12263026, Invitrogen) according to the manufacturer's protocol, to extract mRNA. cDNA was synthesized using Transcriptor-First-Strand-cDNA Synthesis Kit (4897030001, Roche), and then was mixed with primers for IL-1b, IL-8, IL-6, which are inflammatory markers, and GAPDH. Then, cDNA products depending on specific markers were measured using a Light Cycler 480 Real-Time PCR System instrument (Roche) which is real-time PCR. Here, the expression levels of the measured cDNA products were normalized to the expression level of GAPDH, and the expression levels of the markers in the cells cultured under each condition were compared and analyzed. The primers used for qPCR are shown in Table 1.

**[Table 1]**

| Primer | | Sequence information |
|---|---|---|
| Human GAPDH | forward | agc cac atc gct cag aca c |
| | reverse | gcc caa tac gac caa atc c |
| Human IL-1b | forward | TAC CTG TCC TGC GTC TTG AA |
| | reverse | TCT TTG GGT AAT TTT TGG GAT CT |
| Human IL-8 | forward | AGA CAG CAG AGC ACA CAA GC |
| | reverse | ATG GTT CCT TCC GGT GGT |
| Human IL-6 | forward | GAT GAG TAC AAA AGT CCT GAT CCA |
| | reverse | CTG CAG CCA CTG GTT CTG T |

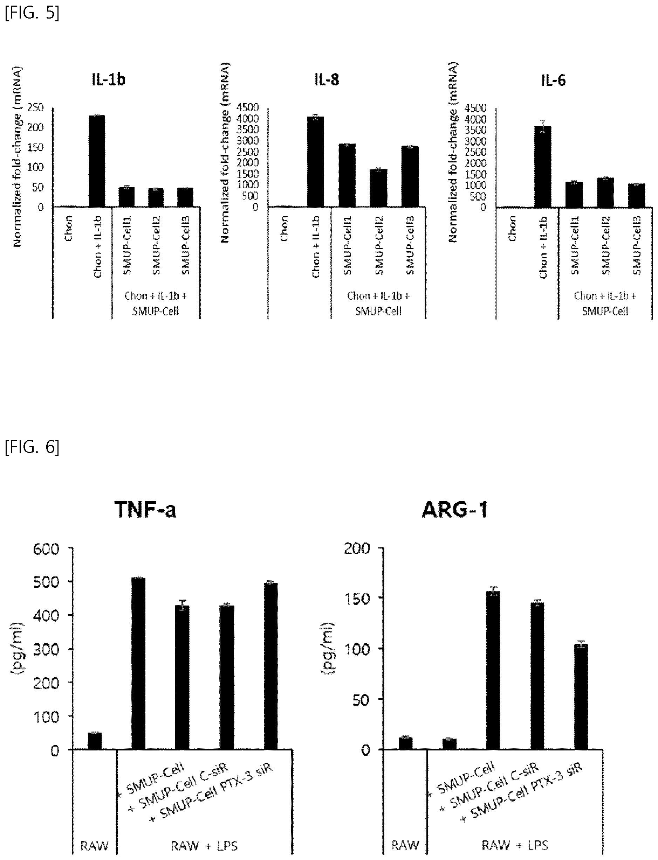

As a result, it was identified that expression levels of IL-1b, IL-8, and IL-6 remarkably increased in group 2), in which the chondrocyte inflammation model was cultured, as compared with group 1) in which chondrocytes were cultured alone. On the contrary, it was identified that expression levels of IL-1b, IL-8, and IL-6 decreased in group 3) in which each of the three types of SMUP-Cell was co-cultured with the chondrocyte inflammation model (FIG. 5).

### Example 3. Identification of anti-inflammatory effect of PTX-3 secreted from mesenchymal stem cells

### Example 3.1. Identification of changes in cytokine expression level in macrophage inflammation model, caused by knockdown of PTX-3 gene in mesenchymal stem cells

To identify correlation between PTX-3 secreted from mesenchymal stem cells and inflammation, cells (SMUP-Cell PTX-3 C-siR) having knocked down PTX-3 expression, obtained by subjecting mesenchymal stem cells to treatment with PTX-3 siRNA, were used. Here, mesenchymal stem cells treated with control siRNA (SMUP-Cell C-siR) were used as a negative control together. The macrophage inflammation model in Example 1.1 and the chondrocyte inflammation model in Example 2.1 were co-cultured with SMUP-Cell or SMUP-Cell PTX-3 C-siR, respectively, and their anti-inflammatory effect was compared and analyzed.

First, using the medium for culture of the macrophage inflammation model, TNF-a, which is an inflammatory cytokine, and arginase-1 (ARG-1), which is an anti-inflammatory cytokine, were measured by ELISA. Specifically, the analysis was performed according to the manufacturer's protocol using Mouse TNF-α ELISA (DY410, R&D Systems) and Mouse Arginase 1 ELISA (ab269541, Abcam). Each culture medium sample after co-culture was diluted between 1 and 50, and analyzed. Then, an OD value for the reaction solution was measured using a VERSA max microplate reader (Molecular Device).

As a result, the expression level of TNF-α decreased and the expression level of ARG-1 increased in the medium, in which the macrophage inflammation model was co-cultured with SMUP-Cell or SMUP-Cell C-siR, as compared with the medium in which the macrophage inflammation model was cultured alone. On the contrary, in the medium in which the macrophage inflammation model was co-cultured with SMUP-Cell PTX-3 siR, the expression level of TNF-a increased and the expression level of ARG-1 decreased. From this, it was identified that expression of PTX-3 was involved in inflammatory response regulation (FIG. 6).

### Example 3.2. Identification of phenotypic changes in macrophage inflammation model following PTX-3 gene knockout in mesenchymal stem cells

To identify phenotypic changes (M1: inflammatory activity, M2: anti-inflammatory activity) in macrophages following LPS treatment-induced inflammation and co-culture with SMUP-Cell, the RAW264.7 cell line, in which inflammation was induced, was fixed, and cell staining was performed with mouse antibodies.

Specifically, the fixed cells were treated with anti-CD11b (Abcam, #ab 128797) and mouse MMR/CD206 (R&D Systems, #AF2535) as primary antibodies, respectively, in refrigeration for 12 to 16 hours, so that the reaction proceeded. Then, treatment with secondary antibodies conjugated with Alexa Fluor 488 (green) and Cy3 (red) was respectively performed for color development, and then the reaction was allowed to proceed at room temperature for 30 minutes. Subsequently, the unreacted reagent was washed off. Then, treatment with Hoechst 33342 (blue, Invitrogen, H3570) was performed for nuclear staining, and the reaction was allowed to proceed for 5 minutes. The final stained cells were photographed using LSM 800 confocal microscopy (Zeiss).

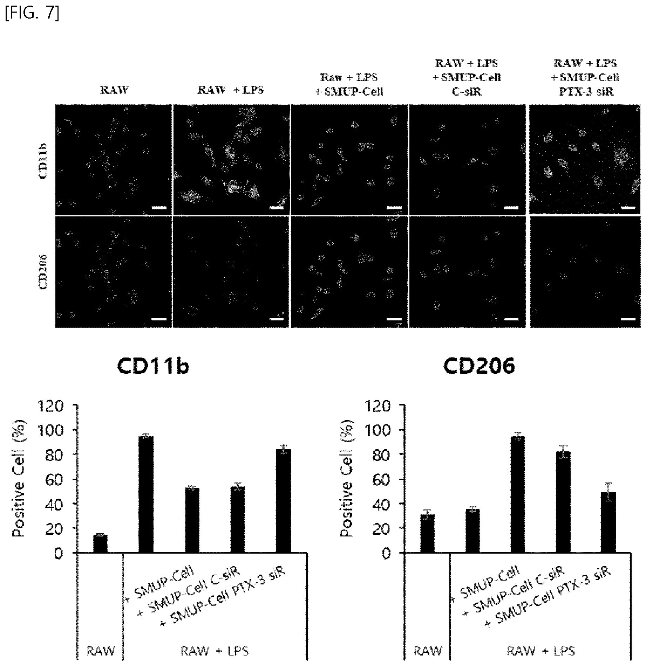

As a result, as illustrated in FIG. 7, the nucleus was stained blue, CD11b (M1 marker) was stained green, and CD206 (M2 marker) was stained red. For the RAW264.7 cell lines in which inflammation was not induced, CD11b (M1 marker) and CD206 (M2 marker) were hardly stained. On the contrary, for the RAW264.7 cell lines in which inflammation was induced by LPS treatment, it was identified that CD 1 1b was stained in most of the cell lines. On the other hand, in a case where SMUP-Cell or SMUP-Cell C-siR was co-cultured with the RAW264.7 cell line in which inflammation was induced, it was identified that a level of staining of CD11b remarkably decreased. In addition, it was identified that CD206 was hardly stained in the RAW264.7 cell line in which inflammation was induced, whereas a level of staining of CD206 increased in the cells co-cultured with SMUP-Cell or SMUP-Cell C-siR. On the contrary, in a case of being co-cultured with SMUP-Cell PTX-3 siR, it was identified that a level of staining of CD11b increased and a level of staining of CD206 decreased. In addition, the same tendency was identified even in the chondrocyte inflammation model.

From this, it was identified that PTX-3 secreted from SMUP-Cell affected macrophage phenotypes and was involved in inflammatory activity regulation.

### Example 3.3. Identification of changes in cytokine expression level in macrophage inflammation model following PTX-3 gene overexpression in mesenchymal stem cells

To identify correlation between PTX-3 secreted from mesenchymal stem cells and inflammation, cells (SMUP-Cell PTX-3 OE) obtained by inducing overexpression (OE) of PTX-3 in mesenchymal stem cells using the CRISPR/CAS9 technique were used. Here, as a negative control, SMUP-Cell C-vec obtained by introduction of only a vector was used. The macrophage inflammation model in Example 1.1 was co-cultured with SMUP-Cell, SMUP-Cell C-vec, and SMUP-Cell PTX-3 OE, respectively, and their anti-inflammatory effect was compared and analyzed.

Using the medium for culture of the macrophage inflammation model, IL-6, which is an inflammatory cytokine, was measured by ELISA. Specifically, the analysis was performed according to the manufacturer's protocol using Mouse IL-6 ELISA (DY406, R&D Systems). Each culture medium sample after co-culture was diluted between 1 and 50, and analyzed. Then, an OD value for the reaction solution was measured using a VERSA max microplate reader (Molecular Device).

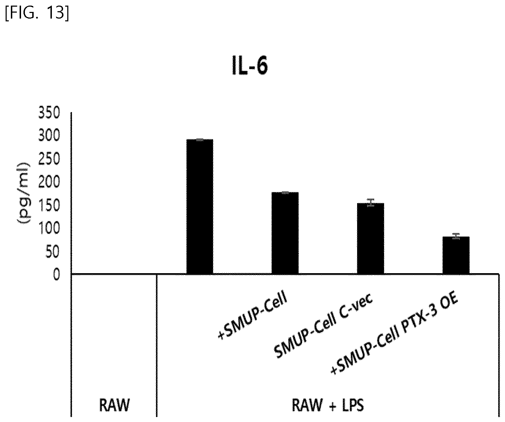

As a result, the expression level of IL-6 similarly decreased in the medium, in which the macrophage inflammation model was co-cultured with SMUP-Cell or SMUP-Cell C-vec, as compared with the medium in which the macrophage inflammation model was cultured alone. On the contrary, in the medium in which the macrophage inflammation model was co-cultured with SMUP-Cell PTX-3 OE, the expression level of IL-6 remarkably decreased as compared with the medium in which the macrophage inflammation model was co-cultured with SMUP-Cell (which was before overexpression). From this, it was identified that expression of PTX-3 was involved in inflammatory response regulation (FIG. 13).

### Example 3.4. Identification of changes in cytokine expression level in chondrocyte inflammation model following PTX-3 gene knockdown in mesenchymal stem cells

The chondrocyte inflammation model in Example 2.1 was co-cultured with SMUP-Cell, SMUP-Cell C-siR, or SMUP-Cell PTX-3 siR, and then expression levels of inflammatory cytokines in the chondrocytes were checked by qPCR. The qPCR was performed in the same manner as in Example 2.2.

As a result, the expression levels of IL-1b, IL-6, IL-8, and TNF-a decreased in the culture medium in which the chondrocyte inflammation model was co-cultured with SMUP-Cell or SMUP-Cell C-siR, as compared with the medium in which the chondrocyte inflammation model was cultured alone. On the contrary, it was identified that high expression levels of IL-1b, IL-6, IL-8, and TNF-a were observed in the medium in which the chondrocyte inflammation model was co-cultured with SMUP-Cell PTX-3 siR, as compared with the medium in which the chondrocyte inflammation model was co-cultured with SMUP-Cell or SMUP-Cell C-siR.

From this, it was identified that expression of PTX-3 in mesenchymal stem cells was involved in inflammatory response regulation in chondrocytes.

### Example 4. Identification of pain reduction effect of PTX-3 secreted from mesenchymal stem cells

For preparation of an arthritis pain-induced rat model, male rats of 150 g to 250 g were anesthetized, and then 2 mg of monoiodoacetate (MIA) was injected using a syringe for intra-articular knee injection. Then, 4 days after MIA injection, injection of mesenchymal stem cells at 2.5×10⁵ cells/25 µl with 1% hyaluronic acid (HA) was performed using a syringe for intra-articular injection. For measurement of pain, an incapacitance test was performed on days 0, 1, 4, 7, 14, 21, and 28 after MIA injection. As a control, a group, into which only 1% HA was injected, was used. In addition, as negative controls, SMUP-Cell treated with PTX-3 siRNA and human umbilical vein endothelial cells (HUVEC) (CRL-1730, ATCC) exhibiting remarkably low PTX-3 expression were used to identify an effect of PTX-3 expression.

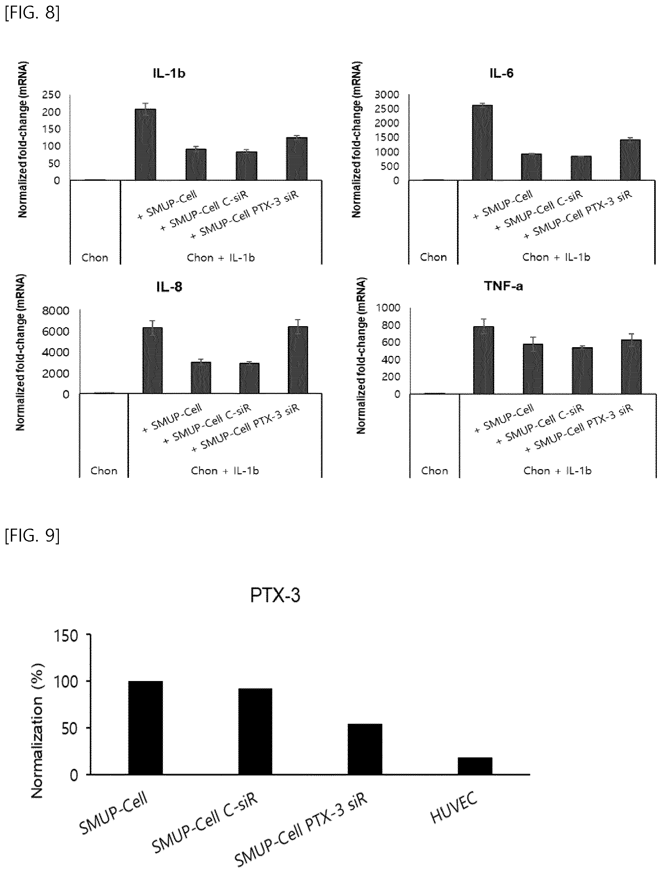

Here, for an expression level of PTX-3 in mesenchymal stem cells, measurement was performed by human PTX-3 ELISA kit for each of the culture media in which the macrophage inflammation model in Example 1.1 was co-cultured with SMUP-Cell, SMUP-Cell C-siR, SMUP-Cell PTX-3 siR, and HUVEC, respectively. Then, the expression level of PTX-3 in SMUP-Cell was set to 100%. This expression level was used to perform conversion and the converted values were graphically expressed for identification. As a result, it was identified that the expression level of PTX-3 decreased in SMUP-Cell PTX-3 siR as compared with SMUP-Cell, and it was identified that the expression level of PTX-3 remarkably decreased in HUVEC as compared with SMUP-Cell PTX-3 siR (FIG. 9).

It was identified whether differences in expression level of PTX-3 in the respective groups caused changes in pain reduction effect. Here, it was identified that for a normal rat, the weight was equally distributed on both hind limbs (ipsilateral/total weight (%) = 50), whereas for the control in which MIA or MIA + HA only were administrated, the pain intensified over time after MIA injection so that the value decreased. In this manner, an animal pain model was prepared. Then, mesenchymal stem cells having different expression levels of PTX-3 were injected into the arthritis pain-induced rats to identify their pain alleviation effect.

As a result, it was identified that in the groups injected with SMUP-Cell and SMUP-Cell C-siR, each of which has a high PTX-3 expression level, the weight bearing value increased from after the injection and was almost restored within 24 days of cell administration. On the contrary, it was identified that in the group transplanted with SMUP-Cell PTX-3 siR, the weight bearing value was not restored and rather decreased even after cell administration. In addition, in the group transplanted with HUVEC, the weight bearing value seemed to be restored in the beginning; however, the value decreased from 10 days after cell administration and no pain reduction tendency was shown.

From this, it was identified that PTX-3 secreted from mesenchymal stem cells was involved in arthritis pain reduction.

### Example 5. Identification of TIMP1 secreted from mesenchymal stem cells and anti-inflammatory effect thereof

To identify correlation between TIMP1 secreted from mesenchymal stem cells and inflammation, cells (SMUP-Cell TIMP1 Knock out, KO) obtained by knocking out TIMP1 expression in mesenchymal stem cells using clustered regularly interspaced short palindromic repeats (CRISPR)/CAS9 system were used to perform a test. Here, as a negative control, SMUP-Cell C-vec obtained by introduction of only a vector was used.

The chondrocyte inflammation model in Example 2 was co-cultured with the mesenchymal stem cells of each group, and then the chondrocyte inflammation model was treated with trypsin to be separated into single cells. mRNA was extracted therefrom and the gene expression pattern of inflammatory cytokines was checked by qPCR. The qPCR was performed in the same manner as in Example 2.2.

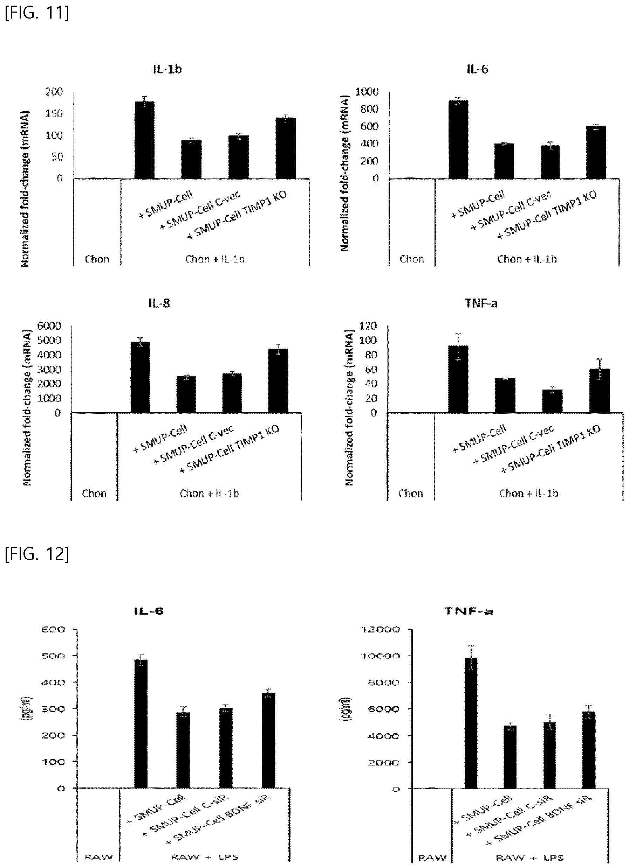

As a result, it was identified that expression levels of IL-1b, IL-6, IL-8, and TNF-α decreased in the chondrocyte inflammatory model co-cultured with SMUP-Cell or SMUP-Cell c-vec, as compared with the normal chondrocytes co-cultured with SMUP-Cell or SMUP-Cell c-vec. On the contrary, it was identified that high expression levels of IL-1b, IL-6, IL-8, and TNF-α were observed in a case where the chondrocyte inflammation model was co-cultured with SMUP-Cell TIMP1 KO, as compared with a case where the chondrocyte inflammation model was co-cultured with SMUP-Cell or SMUP-Cell c-vec. From this, it was identified that expression of TIMP1 in mesenchymal stem cells was involved in inflammatory response regulation (FIG. 11).

### Example 6. Identification of correlation between BDNF secreted from mesenchymal stem cells and anti-inflammation

To identify correlation between BDNF secreted from mesenchymal stem cells and inflammation, cells (SMUP-Cell BDNF C-siR) obtained by subjecting mesenchymal stem cells to treatment with BDNF siRNA for knocking down BDNF expression were used to perform a test. Here, mesenchymal stem cells (SMUP-Cell C-siR) obtained by treatment with control siRNA were used as a negative control together.

The macrophage inflammation model in Example 1 was co-cultured with the mesenchymal stem cells of each group. Then, using each culture medium, expression levels of IL-6 and TNF-a, which are inflammatory cytokines, were measured by a human ELISA kit.

As a result, the expression levels of IL-6 and TNF-a decreased in the medium, in which macrophage inflammation model was co-cultured with SMUP-Cell or SMUP-Cell C-siR, as compared with the medium in which the macrophage inflammation model was cultured alone. On the contrary, high expression levels of IL-6 and TNF-a were observed in the medium, in which the macrophage inflammation model was co-cultured with SMUP-Cell PTX-3 siR, as compared with the medium in which the macrophage inflammation model was co-cultured with SMUP-Cell or SMUP-Cell C-siR. From this, it was identified that expression of BDNF was involved in inflammatory response regulation (FIG. 12).

## Claims

1. A pharmaceutical composition for prevention or treatment of an inflammatory disease or pain, comprising as an active ingredient:
mesenchymal stem cells expressing one or more proteins selected from the group consisting of PTX-3, TIMP 1, and BDNF.

2. The pharmaceutical composition of claim 1, wherein the mesenchymal stem cells express PTX-3.

3. The pharmaceutical composition of claim 1, wherein the mesenchymal stem cells express BDNF.

4. The pharmaceutical composition of claim 1, wherein the mesenchymal stem cells express TIMP1.

5. The pharmaceutical composition of claim 1, wherein the mesenchymal stem cells are prepared by a method including the following steps of:
(1) isolating mesenchymal stem cells having a size of 10 µm or lower;
(2) culturing the isolated mesenchymal stem cells in a culture medium containing calcium under a hypoxic condition of 2% to 5% oxygen; and
(3) subjecting the cultured mesenchymal stem cells to inflammatory stimulation.

6. The pharmaceutical composition of claim 5, wherein the calcium is contained at a concentration of 1.5 to 3.8 mM.

7. The pharmaceutical composition of claim 1, wherein the mesenchymal stem cells are derived from umbilical cord blood, bone marrow, fat, muscle, skin, umbilical cord, amniotic fluid, or tooth.

8. The pharmaceutical composition of claim 1, wherein the inflammatory disease is osteoarthritis, rheumatoid arthritis, atopy, asthma, allergic rhinitis, Alzheimer's disease, graft versus host disease (GVHD), diabetic nephropathy, Crohn's disease, inflammatory bowel disease, post-transplantation rejection, bronchopulmonary dysplasia (BPD), or chronic obstructive pulmonary disease (COPD).

9. The pharmaceutical composition of claim 1, wherein the pain is inflammatory pain.

10. The pharmaceutical composition of claim 9, wherein the inflammatory pain is arthritis pain.

11. The pharmaceutical composition of claim 1, wherein the mesenchymal stem cells exhibit increased expression levels of one or more proteins selected from the group consisting of PTX-3, TIMP1, and BDNF, in an inflammatory environment.

12. A method for obtaining mesenchymal stem cells with improved anti-inflammatory or pain reduction efficacy, the method comprising:
a step of stimulating mesenchymal stem cells so that the mesenchymal stem cells exhibit increased expression of one or more proteins selected from the group consisting of PTX-3, TIMP1, and BDNF.

13. The method of claim 12, wherein the stimulating step includes a step of exposing the mesenchymal stem cells to an inflammatory environment.

14. The method of claim 13, wherein the step of exposing the mesenchymal stem cells to an inflammatory environment includes co-culture with inflammation-induced cells.

15. The method of claim 14, wherein the inflammation-induced cells are cells in which inflammation has been induced by treatment with lipopolysaccharide (LPS), a mitogen, a chemokine, or a cytokine.

16. The method of claim 15, wherein the cells are any one selected from the group consisting of somatic cells, germ cells, and a combination thereof.

17. The method of claim 16, wherein the somatic cells are any one selected from the group consisting of muscle cells, hepatocytes, neurons, fibroblasts, epithelial cells, adipocytes, bone cells, white blood cells, lymphocytes, platelets or mucosal cells, and combinations thereof.

18. The method of claim 12, wherein the mesenchymal stem cells are prepared, before the stimulating step, by a method including the following steps of:
(1) isolating mesenchymal stem cells having a size of 10 µm or lower; and
(2) culturing the isolated mesenchymal stem cells in a culture medium containing calcium under a hypoxic condition of 2% to 5% oxygen.

19. The method of claim 18, wherein the calcium is contained at a concentration of 1.5 to 3.8 mM.

20. A method for preventing or treating an inflammatory disease or pain, comprising a step of administering the pharmaceutical composition of claim 1 to an individual.

21. Use of the pharmaceutical composition of claim 1 for preventing or treating an inflammatory disease or pain.

22. Use of the pharmaceutical composition of claim 1 for preparing a medicament for preventing or treating an inflammatory disease or pain.
